# EUROPEAN PATENT SPECIFICATION

(11) **EP 3 999 476 B1**
(45) Date of publication and mention of the grant of the patent: **14.05.2025**
(21) Application number: 20750737.7
(22) Date of filing: 13.07.2020
(51) Int. Cl.: C02F 3/08, C02F 3/30, C02F 3/22, C02F 3/28

(54) **FIXED FILM BIOLOGICAL ROTATING CONTACTOR WITH HELICOID**
BIOLOGISCHER FESTFILMDREHKONTAKTOR MIT HELIKOID
CONTACTEUR ROTATIF BIOLOGIQUE À FILM FIXE AVEC HÉLICOÏDE

(30) Priority: 15.07.2019 IT 201900011802
(43) Date of publication of application: 25.05.2022
(73) Proprietor: Renda, Roberto, 00715 Roma (IT)
(72) Inventor: Renda, Roberto, 00715 Roma (IT)
(74) Representative: De Gregori, Antonella
(86) International application number: PCT/IB2020/056543
(87) International publication number: WO 2021/009650

(56) References cited:
- EP-A1- 2 412 680
- EP-B1- 2 412 680
- US-A- 3 688 905
- US-A- 4 956 082
- US-A1- 2006 270 024

## Description

### FIELD OF THE INVENTION

The present invention relates to a fixed-biomass biologic reactor with a rotating helicoid contactor.

### STATE OF THE ART

Biologic reactors are known and used in the purification of industrial and/or civil liquid waste (in particular containing water) and in applications where the culture of microorganisms is used to carry out microbiological processes adapted to prepare chemical and pharmaceutical substances; as is known, there are aerobic microbiological processes and anaerobic microbiological processes.

Biologic reactors with fixed biomass (also known as "adhering") are known; the biomass is fixed to a "contactor". An important factor in this type of reactor is the area of the contact surface between biomass and liquid to be treated, in particular the area of the contact surface per unit of volume of the contactor (expressed for example in m2/m3).

Fixed-biomass biologic reactors with rotating contactors are known, for example from European patents no. EP0853067B1 and no. EP2412680B1.

In general, the contactors of known biological reactors have drawbacks linked to mechanical stresses; in particular, the torque moment on the masses involved can cause mechanical breakages.

In general, a further drawback is linked to the proliferation of "undesired" microorganisms which can hinder the growth of and/or the activity of and/or access to the "desired" microorganisms which should determine the operation of the reactor.

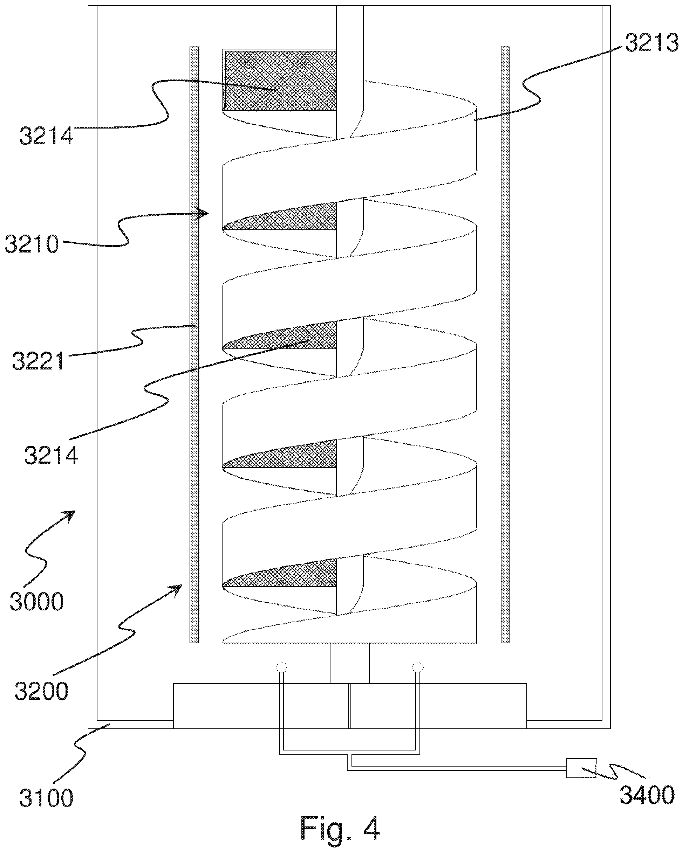

From document US2006/270024 a bioreactor is known for the treatment of municipal or industrial contaminated water in which there is a conical "helicoid" (called in this document "filling body" and shown in Fig. 4) covered with a microbiotic mix, home to biological reactions. According to this solution, the microbiotic mix consists of two different types of microorganisms: the microorganisms of a first type are adapted to emit light and the microorganisms of a second type are adapted to receive the light emitted by the others and use it to make photosynthetic reactions. Again according to this solution (see paragraph 0057), in the upper part of the "helicoid" there are microorganisms and there is biofilm formation and in the lower part there is a catalyst, in particular a photocatalyst (for example titanium oxide as indicated in paragraph 0054), which avoids the formation of biofilm and serves to start the process of degradation of polluting substances which is then completed by microorganisms; moreover, the catalyst or photocatalyst can also be positioned on the walls of a basket which surrounds the "helicoid". This document also discloses a solution with cylindrical "helicoid" (Fig. 6) which is similar to the solution with conical "helicoid" (Fig. 4) as regards the positions of microorganisms and catalyst. It is therefore understood that the microorganisms must be fixed to the helicoid in a suitable position, i.e. in such a way that they are separated from the catalyst or photocatalyst.

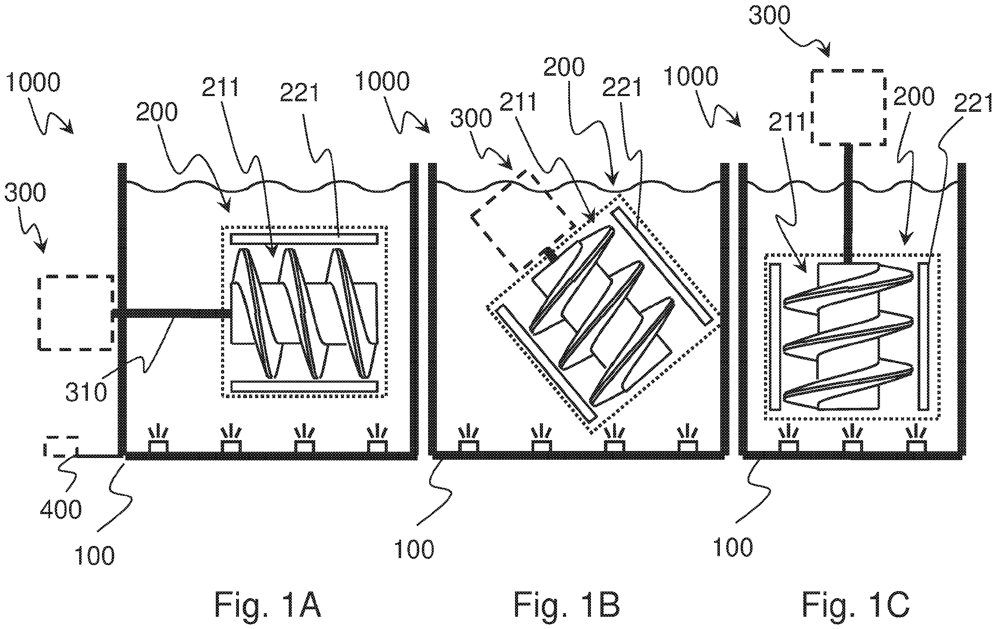

The document EP2412680B1 discloses a fixed-biomass biologic reactor with rotating cylindrical contactor with a horizontal axis; the contactor comprises walls which extend radially from the centre of the contactor and which define angular sectors in which a plurality of filling bodies covered with biomass are arranged. In cross section (the section is uniform moving along the direction of the rotation axis of the contactor - see Fig. 1), it is seen that the walls form a sort of turbine which is rotated by gas bubbles which are generated on the bottom of the reactor at a given distance from the contactor. It is understood that the bubbles remain in contact with the filling bodies for a relatively short time (corresponding to the time taken by the contactor to make half a turn) and substantially always with the same filling bodies, i.e. those that are close to the walls.

### ABSTRACT

The general object of the present invention is to improve the known art.

This general purpose and other specific purposes are substantially achieved thanks to what is expressed in the appended claims which form an integral part of the present description.

The idea behind the present invention is to use a rotating helicoid in the contactor, in particular shaped like an "Archimedes screw", and a plurality of filling bodies on which biomass is fixed; in particular, the helicoid is located inside a tube (closed laterally, but not at the ends) of the contactor; in particular, the filling bodies are placed in one or more compartments of the helicoid and/or in one or more compartments of the tube in the inner space of the tube not occupied by the helicoid.

A "helicoid" is a surface generated by the rotation and at the same time by the translation of a curve along a line, where said line constitutes the axis of the helicoid. In other words, a helicoid is a surface formed by screwings having a given diameter around an axis, spaced by a pitch defined by the translation motion in the direction defined by the axis. In particular, the surfaces of two consecutive screwings define at least one free space, that is a space not occupied by the helicoid. For the purposes of the present invention, the helicoid can have a variable pitch and/or a not constant diameter.

### LIST OF FIGURES

The present invention shall become more readily apparent from the detailed description that follows to be considered together with the accompanying drawings in which:
Fig. 1 shows very schematically three variants of a first example of reactor according to the present invention,
Fig. 2 shows very schematically three variants of a second example of reactor according to the present invention,
Fig. 3 schematically shows a longitudinal section view of a third example of reactor according to the present invention,
Fig. 4 schematically shows a partial side view of a third example of reactor according to the present invention, and
Fig. 5 show two details of the reactor of Fig. 3 and Fig. 4.

As can be easily understood, there are various ways of practically implementing the present invention which is defined in its main advantageous aspects in the appended claims.

### DETAILED DESCRIPTION

The biologic reactor according to the present invention can have various applications according to the specific embodiment. It can find application for example in the purification of industrial and/or civil liquid waste and in the preparation of chemical and pharmaceutical substances. It can be used both to carry out aerobic microbiological processes and to carry out anaerobic microbiological processes.

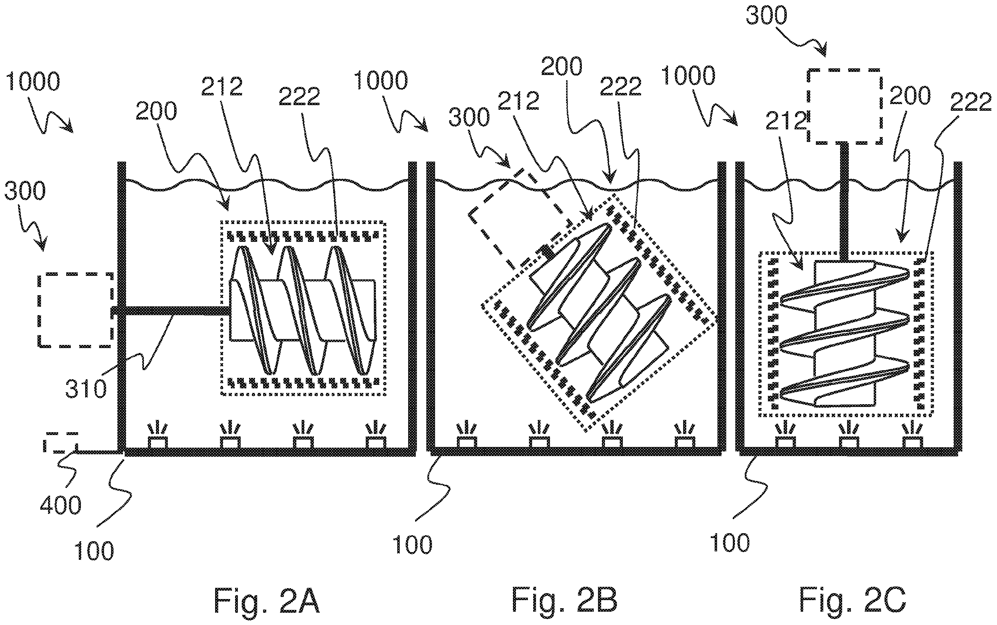

In general and as shown in figures 1 and 2, a reactor 1000 comprises a tub 100 adapted to contain a liquid to be processed, and a contactor 200 placed in the tub 100. The tub 100 can be opened or closed according to the desired process. The contactor 200 contains biomass and, during the operation of the reactor 1000, it is traversed by the flow of the liquid contained in the tub 100; in this way, the flowing liquid comes into contact with the biomass and is subject to microbiological processes by the biomass. The contactor 200 comprises a helicoid adapted to rotate during the operation of the reactor 1000, and a tube in which the helicoid is located. As shown in the attached figures, this helicoid is shaped like an "Archimedes screw". As will be understood hereinafter, the contactor of a reactor according to the present invention could comprise multiple tubes. Typically, the helicoid of the contactor 200 is completely immersed in the liquid of the tub 100.

Typically, a reactor according to the present invention is equipped with multiple contactors or with a multiple-helix contactor; however, simple examples with a single helicoid contactor are described here.

In the embodiments of Fig. 1 and Fig. 2, the axis of the helicoid (i.e. the rotation axis of the helicoid) is positioned in such a way as to form an angle with respect to a horizontal plane, i.e. it is fixed, during the operation of the reactor. Preferably but not necessarily, the angle is a right angle, i.e. 90°.

In the embodiments of Fig. 1 and Fig. 2, the axis of the helicoid and the axis of the tube coincide; in practical embodiments, the axes may not even exactly coincide.

In the embodiments of Fig. 1 and Fig. 2, the length of the helicoid and the length of the tube coincide; alternatively, the tube could be a little longer than the helicoid at one or at each end, and/or a little shorter than the helicoid at one or at each end.

In Fig. 1A and Fig. 1B and Fig. 1C, the side wall of the tube 221 is entirely full, i.e. there can be no flow of liquid through this wall. Alternatively, the tube 221 could have a side wall partially or entirely with openings, for example pierced.

In Fig. 2A and Fig. 2B and Fig. 2C, the side wall of the tube 222 is entirely with openings, i.e. there is flow of liquid through this wall; the tube 222 can be, for example, an entirely full wall tube with side holes or an entirely mesh tube. Alternatively, the tube 222 could have a partially or entirely full side wall.

In Fig. 1A and Fig. 1B and Fig. 1C, the helicoid 211 and the tube 221 are separated from each other. Typically, the helicoid 211 is adapted to rotate and the tube 221 is adapted to remain fixed.

In Fig. 2A and Fig. 2B and Fig. 2C, the helicoid 212 and the tube 222 are joined together. Typically, the helicoid 212 and the tube 222 are adapted to rotate integrally. In Fig. 1A, the angle of the axis of the helicoid 211 is about 0°.

In Fig. 1B, the angle of the axis of the helicoid 211 is about 45°.

In Fig. 1C, the angle of the axis of the helicoid 211 is about 90°.

In Fig. 2A, the angle of the axis of the helicoid 212 is about 0°.

In Fig. 2B, the angle of the axis of the helicoid 212 is about 45°.

In Fig. 2C, the angle of the axis of the helicoid 212 is about 90°.

The reactor 1000 of Fig. 1 and Fig. 2 can comprise a motor 300 adapted to rotate at least the helicoid; the motor 300 is drawn with a dotted line because its presence is advantageous, but not essential and depends on the specific embodiment of the present invention. By way of non-limiting example, in Fig. 1A and Fig. 2A the motor 300 is completely external to the tub 100, in Fig. 1B and Fig. 2B the motor 300 is partially internal and partially external to the liquid of the tub 100, in Fig. 1C and Fig. 2C the motor 300 is completely external to the liquid of the tub 100. It should be noted that a mechanical transmission 310 can be placed between the motor shaft 300 and the helicoid shaft.

The reactor 1000 of Fig. 1 and Fig. 2 can comprise a generator 400 of gas bubbles (for example air) adapted to emit gas bubbles in the liquid of the tub 100; the generator 400 is designed with a continuous line because its presence is advantageous and very typical, although not essential, and depends on the specific embodiment of the present invention. The generator 400 can be adapted to emit gas bubbles in the tub 100 and/or in the tube of the contactor 200.

The generator 400 can be adapted to rotate at least the helicoid due to the gas bubbles emitted. In this case, for example, the generator 400 can be adapted to emit gas bubbles in the vicinity of a first end of the helicoid and/or of the tube.

The simultaneous presence of both a motor and a gas bubble generator may be envisaged.

As previously mentioned, the contactor 200 contains biomass. According to the present invention the contactor 200 contains a plurality of filling bodies and the biomass is fixed only on the filling bodies; the filling bodies are in one or more compartments of the helicoid and/or in one or more compartments of the tube in the inner space of the tube not occupied by the helicoid. According to a non claimed second possibility, the biomass can also be fixed on the helicoid (and/or the accessories thereof). According to a non claimed third possibility, the biomass can also be fixed on the tube (and/or the accessories thereof). These three possibilities can be combined between them.

According to some advantageous embodiments of the present invention, the degradation of polluting substances is taken over only by the biomass fixed on the filling bodies, in particular to the microorganisms contained. In this way, for example, the "regeneration" of the reactor can be achieved very simply by replacing the filling bodies.

Typically, the filling bodies can be granules or pellets that act as a support for biomass. "Granules" refers to small bodies having irregular shape and, in general, shape and size that are different from each other; their size can range from for example a few millimetres to for example ten millimetres. Their surface can be porous.

"Pellet" refers to a body having a shape typically larger than a granule, for example with size that can range from about ten millimetres to for example a hundred millimetres. In general, they can have various forms, typically hollow (for example with a filling index greater than 80% or 90%), so as to allow them to be traversed by a fluid and increase the surface available to the interaction between biomass and fluid. Their surface can be porous. Pellets of this kind are produced for example by the company Ecoplast Srl - see the link "https://www.ecoplast.it/site/prodotti".

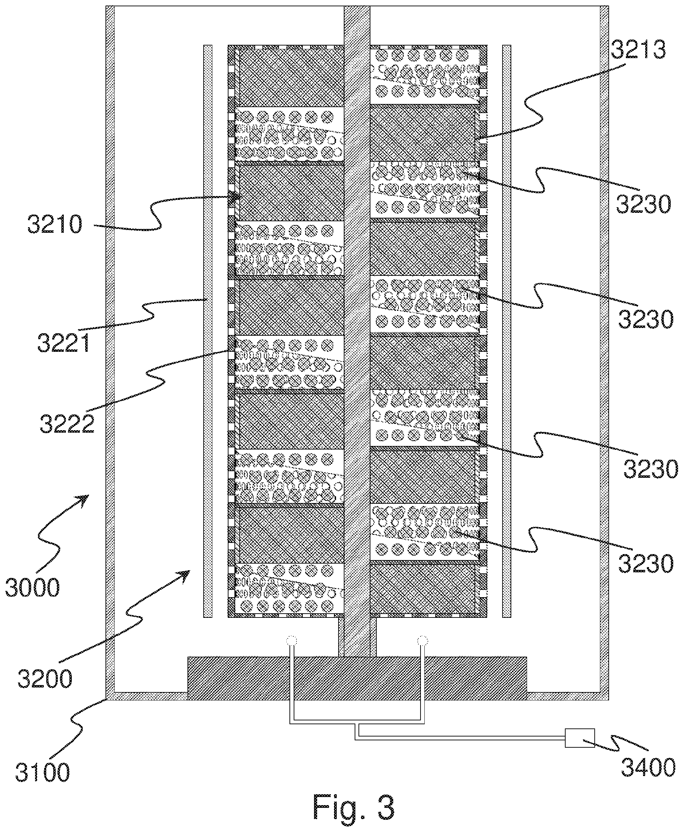

The reactor 3000 of Fig. 3 and Fig. 4 is similar to the reactor of Fig. 2C.

The reactor 3000 comprises a tub 3100 adapted to contain a liquid to be processed, and a contactor 3200 placed in the tub 3100.

The contactor 3200 comprises a helicoid 3210 adapted to rotate during the operation of the reactor 3000, an inner tube 3222 joined to the helicoid 3210, and an outer tube 3221 (in particular coaxial and with a slightly larger diameter) separated from the helicoid 3210 and adapted to remain fixed during operation of the reactor 3000. The contactor 3200, in particular the helicoid 3210, is completely immersed in the liquid of the tub 3100.

The axis of the helicoid 3210 (i.e. the rotation axis of the helicoid) is placed in such a way as to form an angle of about 90° with respect to a horizontal plane, i.e. it is fixed. The axes of helicoid 3210, of the tube 3221 and of the tube 3222 coincide.

The length of the helicoid 3210, of the tube 3221 and of the tube 3222 coincide.

The side wall of the outer tube 3221 is entirely full.

The side wall of the inner tube 3222 is entirely with openings.

The angle of the axis of the helicoid 3210 is about 90°.

The reactor 3000 can comprise a motor 300 adapted to rotate the helicoid 3210 and the inner tube 3222.

The reactor 3000 comprises a generator 3400 of gas bubbles (for example air) adapted to emit gas bubbles in the tub 3100 in the vicinity of the lower end of the helicoid 3210, of the tube 3221 and of the tube 3222. Said gas bubbles can contribute to rotating the helicoid 3210 and the inner tube 3222.

In Fig. 3, filling bodies 3230 on which biomass is fixed are schematized; the bodies 3230 are placed in compartments of the helicoid 3210.

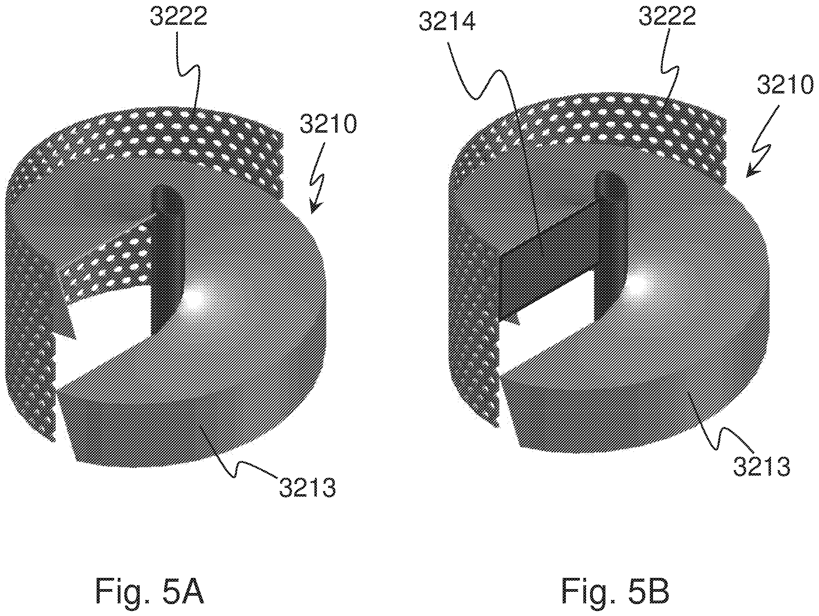

As can be seen particularly well in Fig.5A, the helicoid 3210 can have a circumferential edge 3213 (which can be full or pierced) which extends (at least in part) axially; the edge 3213 is preferably full to contain both the filling bodies and the gas bubbles, but it can also be with openings (for example pierced).

As can be seen particularly well in Fig.5B, the helicoid 3210 can have a plurality of septa 3214 which extend (at least in part) axially and radially; the septa 3214 are preferably full to contain both the filling bodies and the gas bubbles, but they can also be with openings (for example pierced).

It is advantageous that the circumferential edge and the septa are present in combination. Each of these elements serves to constrain the filling bodies to the "helicoid", however avoiding a fixed connection; in other words, the filling bodies may have small movements with respect to the "helicoid" during the operation of the reactor which are very useful in exploiting the active biomass at best.

In general, the active biomass can be also fixed (in addition to the filling bodies 3230) to one or more of the following components:
- the rolling surface of the helicoid 3210 of the contactor 3200
- the edge 3213 of the helicoid 3210 of the contactor 3200
- the septa 3214 of the helicoid 3210 of the contactor 3200
- the inner tube 3222 of the contactor 3200
- the outer tube 3221 of the contactor 3200

In the example of Fig. 3 and Fig. 4, during the operation of the reactor 3000, there is flow of liquid and gas bubbles from a lower end of the outer tube 3221 to an upper end of the outer tube 3221.

The flowing liquid is subject to microbiological processes by the biomass present in the contactor 3200.

The gas bubbles tend to rise upwards, favouring the rotation of the contactor 3200 and the components thereof; the rotation of the helicoid 3210 moves the liquid downwards or upwards according to the direction of the helicoid.

## Claims

1. Reactor (1000) comprising a tub (100) adapted to contain a liquid and a contactor (200) placed in said tub (100) and adapted to be traversed by a flow of said liquid, wherein said contactor (200) contains a plurality of filling bodies on which biomass is fixed, said biomass being adapted to carry out a microbiological process on the flowing liquid, wherein said contactor (200) comprises:
- a helicoid (211, 212) adapted to rotate, and
- a tube (221, 222) in which the helicoid is located (211, 212);
**characterised in that** the biomass is fixed only on the filling bodies, the filling bodies being placed in one or more compartments of the helicoid and/or in one or more compartments of the tube in the inner space of the tube not occupied by the helicoid.

2. Reactor (1000) according to claim 1, wherein said helicoid (211, 212) and said tube (221, 222) are joined together or separated from each other.

3. Reactor (3000) according to claim 1 or 2, wherein said contactor (3200) comprises a helicoid (3210), an inner tube (3222) joined to said helicoid (3210) and an outer tube (3221) separated from said helicoid (3210).

4. Reactor (1000) according to any one of the preceding claims, comprising a motor (300) adapted to rotate said helicoid (211, 212).

5. Reactor (1000) according to any one of the preceding claims, comprising a gas bubble generator (400) adapted to emit gas bubbles in said liquid in said tub (100).

6. Reactor (1000) according to claim 5, wherein said generator (400) is adapted to rotate said helicoid (211, 212) due to the gas bubbles emitted.

7. Reactor (1000) according to any one of the preceding claims, wherein the axis of said helicoid (211, 212) is positioned so as to form an angle with respect to a horizontal plane, preferably a right angle.

8. Reactor (1000) according to any one of the preceding claims, wherein said helicoid (211, 212) is adapted to be totally immersed in said liquid.

9. Reactor (3000) according to any one of the preceding claims, wherein said helicoid (3210) has a circumferential edge (3213) which extends axially.

10. Reactor (3000) according to any one of the preceding claims, wherein said helicoid (3210) has a plurality of septa (3214) which extend radially.

11. Reactor (3000) according to any one of the preceding claims, wherein said contactor (3200) is made so that there is flow of said liquid from a first end of a tube thereof (3221) to a second end of a tube thereof (3221).

## Patentansprüche

1. Reaktor (1000), umfassend eine Wanne (100), die geeignet ist, eine Flüssigkeit zu enthalten, und einen Kontaktor (200), der in der Wanne (100) angeordnet ist und geeignet ist, von einem Strom der Flüssigkeit durchströmt zu werden, wobei der Kontaktor (200) eine Vielzahl von Füllkörpern enthält, auf denen Biomasse fixiert ist, wobei die Biomasse geeignet ist, einen mikrobiologischen Prozess an der strömenden Flüssigkeit auszuführen, wobei der Kontaktor (200) Folgendes umfasst:
- ein drehbares Helikoid (211, 212) und
- ein Rohr (221, 222), in dem das Helikoid (211, 212) angeordnet ist;
**dadurch gekennzeichnet, dass** die Biomasse nur auf den Füllkörpern fixiert ist, wobei die Füllkörper in einem oder mehreren Fächern des Helikoid und/oder in einem oder mehreren Fächern des Rohrs im Innenraum des Rohrs, der nicht von dem Helikoid eingenommen wird, angeordnet sind.

2. Reaktor (1000) nach Anspruch 1, wobei das Helikoid (211, 212) und das Rohr (221, 222) miteinander verbunden oder voneinander getrennt sind.

3. Reaktor (3000) nach Anspruch 1 oder 2, wobei der Kontaktor (3200) ein Helikoid (3210), ein mit dem Helikoid (3210) verbundenes inneres Rohr (3222) und ein von dem Helikoid (3210) getrenntes äußeres Rohr (3221) umfasst.

4. Reaktor (1000) nach einem der vorstehenden Ansprüche, umfassend einen Motor (300), der geeignet ist, das Helikoid (211, 212) in Drehung zu versetzen.

5. Reaktor (1000) nach einem der vorstehenden Ansprüche, der einen Gasblasengenerator (400) umfasst, der geeignet ist, Gasblasen in die Flüssigkeit in der Wanne (100) abzugeben.

6. Reaktor (1000) nach Anspruch 5, wobei der Generator (400) geeignet ist, das Helikoid (211, 212) aufgrund der abgegebenen Gasblasen in Drehung zu versetzen.

7. Reaktor (1000) nach einem der vorstehenden Ansprüche, wobei die Achse des Helikoids (211, 212) so angeordnet ist, dass sie einen Winkel, vorzugsweise einen rechten Winkel, in Bezug auf eine horizontale Ebene bildet.

8. Reaktor (1000) nach einem der vorstehenden Ansprüche, wobei das Helikoid (211, 212) geeignet ist, vollständig in die Flüssigkeit eingetaucht zu werden.

9. Reaktor (3000) nach einem der vorstehenden Ansprüche, wobei das Helikoid (3210) einen umlaufenden Rand (3213) aufweist, der sich axial erstreckt.

10. Reaktor (3000) nach einem der vorstehenden Ansprüche, wobei das Helikoid (3210) eine Vielzahl von Septen (3214) aufweist, die sich radial erstrecken.

11. Reaktor (3000) nach einem der vorstehenden Ansprüche, wobei der Kontaktor (3200) so ausgebildet ist, dass die Flüssigkeit von einem ersten Ende eines Rohres (3221) davon zu einem zweiten Ende eines Rohres (3221) davon fließt.

## Revendications

1. Réacteur (1000) comprenant une cuve (100) adaptée pour contenir un liquide et un contacteur (200) placé dans ladite cuve (100) et adapté pour être traversé par un écoulement dudit liquide, dans lequel ledit contacteur (200) contient une pluralité de corps de remplissage sur lesquels une biomasse est fixée, ladite biomasse étant adaptée pour effectuer un processus microbiologique sur le liquide en écoulement, dans lequel ledit contacteur (200) comprend :
- un hélicoïde (211, 212) pouvant tourner, et
- un tube (221, 222) dans lequel se trouve l'hélicoïde (211, 212) ;
**caractérisé en ce que** la biomasse est fixée uniquement sur les corps de remplissage, les corps de remplissage étant placés dans un ou plusieurs compartiments de l'hélicoïde et/ou dans un ou plusieurs compartiments du tube dans l'espace intérieur du tube non occupé par l'hélicoïde.

2. Réacteur (1000) selon la revendication 1, dans lequel ledit hélicoïde (211, 212) et ledit tube (221, 222) sont reliés entre eux ou séparés l'un de l'autre.

3. Réacteur (3000) selon la revendication 1 ou 2, dans lequel ledit contacteur (3200) comprend un hélicoïde (3210), un tube intérieur (3222) relié audit hélicoïde (3210) et un tube extérieur (3221) séparé dudit hélicoïde (3210).

4. Réacteur (1000) selon l'une quelconque des revendications précédentes, comprenant un moteur (300) pouvant faire tourner ledit hélicoïde (211, 212).

5. Réacteur (1000) selon l'une quelconque des revendications précédentes, comprenant un générateur de bulles de gaz (400) adapté pour émettre des bulles de gaz dans ledit liquide dans ladite cuve (100).

6. Réacteur (1000) selon la revendication 5, dans lequel ledit générateur (400) est adapté pour faire tourner ledit hélicoïde (211, 212) sous l'effet des bulles de gaz émises.

7. Réacteur (1000) selon l'une quelconque des revendications précédentes, dans lequel l'axe dudit hélicoïde (211, 212) est positionné de manière à former un angle par rapport à un plan horizontal, de préférence un angle droit.

8. Réacteur (1000) selon l'une quelconque des revendications précédentes, dans lequel ledit hélicoïde (211, 212) est adapté pour être totalement immergé dans ledit liquide.

9. Réacteur (3000) selon l'une quelconque des revendications précédentes, dans lequel ledit hélicoïde (3210) présente un bord circonférentiel (3213) qui s'étend axialement.

10. Réacteur (3000) selon l'une quelconque des revendications précédentes, dans lequel ledit hélicoïde (3210) présente une pluralité de septums (3214) qui s'étendent radialement.

11. Réacteur (3000) selon l'une quelconque des revendications précédentes, dans lequel ledit contacteur (3200) est réalisé de telle sorte que ledit liquide s'écoule d'une première extrémité de son tube (3221) vers une seconde extrémité de son tube (3221).
